# EUROPEAN PATENT SPECIFICATION

(11) **EP 3 521 447 B1**
(45) Date of publication and mention of the grant of the patent: **11.08.2021**
(21) Application number: 17855832.6
(22) Date of filing: 19.09.2017
(51) Int. Cl.: C12Q 1/6806, C12N 15/10

(54) **NUCLEIC ACID EXTRACTION METHOD AND KIT USING SAME**
NUKLEINSÄUREEXTRAKTIONSVERFAHREN UND KIT
PROCÉDÉ D'EXTRACTION D'ACIDE NUCLÉIQUE ET KIT L'UTILISANT

(30) Priority: 30.09.2016 JP 2016193987
(43) Date of publication of application: 07.08.2019
(73) Proprietor: Eiken Kagaku Kabushiki Kaisha, Taito-ku Tokyo 110-8408 (JP)
(72) Inventor: SATO, Masaki, Shimotsuga-gun Tochigi 329-0114 (JP); YONEKAWA, Toshihiro, Shimotsuga-gun Tochigi 329-0114 (JP)
(74) Representative: Boult Wade Tennant LLP
(86) International application number: PCT/JP2017/033741
(87) International publication number: WO 2018/061877

(56) References cited:
- WO-A1-2008/121927
- JP-A- 2004 073 193
- JP-A- 2004 340 839
- JP-A- 2008 529 509
- JP-A- 2010 523 943
- JP-A- 2013 516 185
- JP-A- 2014 030 364
- JP-A- 2014 030 364
- US-A1- 2012 295 328

### SEQUENCE LISTING

<110> Eiken Chemical CO., LTD
<120> METHOD FOR EXTRACTING NUCLEIC ACIDS AND KIT FOR THE SAME
<130> FP17-0614-00
<150> JP 2016-193987
   <151> 2016-09-30
<160> 15
<170> PatentIn version 3.5
<210> 1
   <211> 21
   <212> DNA
   <213> Artificial Sequence
<220>
   <223> S.P forward primer
<400> 1
   acgcaatcta gcagatgaag c 21
<210> 2
   <211> 21
   <212> DNA
   <213> Artificial Sequence
<220>
   <223> S.P reverse primer
<400> 2
   tgtttggttg gttattcgtg c 21
<210> 3
   <211> 25
   <212> DNA
   <213> Artificial Sequence
<220>
   <223> S.P probe
<400> 3
   tttgccgaaa acgcttgata caggg 25
<210> 4
   <211> 19
   <212> DNA
   <213> Artificial Sequence
<220>
   <223> B.P forward primer
<400> 4
   atcaagcacc gctttaccc 19
<210> 5
   <211> 21
   <212> DNA
   <213> Artificial Sequence
<220>
   <223> B.P reverse primer
<400> 5
   ttgggagttc tggtaggtgt g 21
<210> 6
   <211> 22
   <212> DNA
   <213> Artificial Sequence
<220>
   <223> B.P probe
<400> 6
   aatggcaagg ccgaacgctt ca 22
<210> 7
   <211> 20
   <212> DNA
   <213> Artificial Sequence
<220>
   <223> S.C forward primer
<400> 7
   ggactctgga catgcaagat 20
<210> 8
   <211> 21
   <212> DNA
   <213> Artificial Sequence
<220>
   <223> S.C reverse primer
<400> 8
   atacccttct taacacctgg c 21
<210> 9
   <211> 30
   <212> DNA
   <213> Artificial Sequence
<220>
   <223> S.C probe
<400> 9
   cccttcagag cgttttctct aaattgatac 30
<210> 10
   <211> 23
   <212> DNA
   <213> Artificial Sequence
<220>
   <223> ADV forward primer
<400> 10
   gccaccgtgg ggtttctaaa ctt 23
<210> 11
   <211> 25
   <212> DNA
   <213> Artificial Sequence
<220>
   <223> ADV reverse primer
<400> 11
   gccgcagtgg tcttacatgc acatc 25
<210> 12
   <211> 29
   <212> DNA
   <213> Artificial Sequence
<220>
   <223> ADV probe
<400> 12
   tgcaccaggc cccggctcag gtactccga 29
<210> 13
   <211> 29
   <212> DNA
   <213> Artificial Sequence
<220>
   <223> FluA forward primer
<220>
   <221> misc_feature
   <222> (3)..(3)
   <223> m is a or c
<400> 13
   ccmaggtcga aacgtaygtt ctctctatc 29
<210> 14
   <211> 32
   <212> DNA
   <213> Artificial Sequence
<220>
   <223> FluA reverse primer
<400> 14
   tgacagraty ggtcttgtct ttagccaytc ca 32
<210> 15
   <211> 22
   <212> DNA
   <213> Artificial Sequence
<220>
   <223> FluA probe
<400> 15
   atytcggctt tgagggggcc tg 22

## Description

### Technical Field

The present invention relates to a nucleic acid extraction method and a kit used therefor.

### Background Art

The BOOM method is one of the methods for extracting nucleic acid from biological samples (for example, see Patent Literature 1). In this method, nucleic acid is adsorbed on silica particles and is isolated from the biological sample, in the presence of a chaotropic compound, which is a substance that robs hydration water from biopolymers such as nucleic acid, and destabilize the higher-order structure of the biopolymers.

An example of method using the BOOM method is a method comprising lysing the biological sample and fixing silica and the like and the nucleic acid, in the presence of a chaotropic compound and/or an alkanol, wherein the fixing of the nucleic acid is performed within a temperature range of 36 to 75°C (for example, see Patent Literature 2).

### Citation List

### Patent Literature

Patent Literature 1: Japanese Unexamined Patent Publication No. H2-289596
Patent Literature 2: Japanese Unexamined Patent Publication No. 2008-529509

### Summary of Invention

### Technical Problem

In order to extract nucleic acid, a lysing step is necessary to break the membrane structure of the cell and release the contents outside the cell. As a lysing method, an enzymatic lysis method using proteolytic enzymes is widely used in commercial nucleic acid extraction kits. Moreover, when extracting nucleic acid from cells having special cell walls such as gram-positive bacteria or fungi among microorganisms, a pre-pretreatment with a cell wall degrading enzyme such as lysozyme and zymolyase is separately required. Therefore, depending on the type of biological material, the corresponding kit or protocol is generally different.

Moreover, many of the kits using the enzymatic lysis method take most of the time on the lysing step, relative to the time for all of the steps for nucleic acid extraction. Moreover, a centrifugal separation step is also often needed. Therefore, it is not suitable for applications where promptness is required.

The present invention was made in consideration of these issues, therefore, the aim of the present invention is to provide a method for extracting nucleic acid and a kit used therefor, that can reduce the amount of time necessary to extract nucleic acid from a biological sample.

### Solution to Problem

The present invention provides a method for extracting nucleic acid from biological samples, comprising steps of: (i) mixing a biological sample, an anionic surfactant, namely lithium dodecyl sulfate (LDS), and a chaotropic compound to obtain a lysate; (ii) mixing the lysate, an alkanol having a boiling point higher than 75°C, namely 1-propanol, a chaotropic compound and silica particles to adsorb the nucleic acid on the silica particles; (iii) washing the silica particles having the nucleic acid adsorbed thereon with a washing liquid; and (iv) eluting the nucleic acid adsorbed on the silica particles with an eluent, wherein at least steps (i) and (ii) are performed at a temperature higher than 75°C.

In the above method, step (iv) is preferably performed at a temperature higher than 75°C.

The alkanol having a boiling point higher than 75°C is 1-propanol.

The anionic surfactant is lithium dodecyl sulfate.

The chaotropic compound may be a guanidinium salt.

The present invention also provides a nucleic acid extraction kit comprising a first lysis solution comprising an anionic surfactant, namely LDS, a second lysis solution comprising a chaotropic compound and an adsorbing liquid comprising an alkanol having a boiling point higher than 75°C, namely 1-propanol, a chaotropic compound and silica particles.

In the kit, the alkanol having a boiling point higher than 75°C is 1-propanol.

In the kit, the anionic surfactant is lithium dodecyl sulfate.

In the kit, the chaotropic compound may be a guanidinium salt.

### Advantageous Effects of Invention

According to the present invention, a method for extracting nucleic acid and a kit used therefor, that can reduce the amount of time necessary to extract nucleic acid from a biological sample, can be provided.

### Brief Description of Drawings

Figure 1 shows the amount of nucleic acid extracted by an automated nucleic acid extractor: (a) a sample containing S. pneumoniae; (b) a sample containing B. pertussis; (c) a sample containing S. cerevisiae; (d) a sample containing human adenovirus 2; and (e) a sample containing influenza A virus.
Figure 2 shows the amount of nucleic acid extracted by an automated nucleic acid extractor: (a) a sample where a specimen containing S. pneumoniae is added to a specimen from a healthy subject; and (b) a sample where a specimen containing human adenovirus 2 is added to a specimen from a healthy subject.
Figure 3 shows the relation between elution temperature and the amount of nucleic acid extracted: (a) a sample containing S. pneumoniae; and (b) a sample containing influenza A virus.

### Description of Embodiments

The following describes in detail the embodiments of the present invention. However, the present invention is not limited to the following embodiments, but rather it is defined in the appended claims.

The method for extracting nucleic acid from a biological sample, according to the present embodiment, comprises (i) mixing a biological sample, an anionic surfactant and a chaotropic compound to obtain a lysate.

The lysate is obtained, for example, by adding and mixing the biological sample, a first lysis solution comprising the anionic surfactant and a second lysis solution comprising the chaotropic compound, in a microtube placed on a heat block, then heating it.

The biological sample, the first lysis solution comprising the anionic surfactant and the second lysis solution comprising the chaotropic compound may be mixed at the same time, or either the first lysis solution comprising the anionic surfactant or the second lysis solution comprising the chaotropic compound may be mixed with the biological sample to obtain a mixed liquid, then the mixed liquid may be mixed with the other solution.

In this description, "biological sample" refers to a specimen that are subjected to the extraction of nucleic acid, and, specifically, is a part or the entirety of the cell, tissue, or organ of viruses, phages, bacteria, fungi and living organisms, and includes specimens collected directly from living body as well as those obtained from the environment such as water, soil or air. Examples of biological sample include blood, throat swabs, nasopharyngeal swabs, sputum, spinal fluid, urine, feces and saliva.

The nucleic acid may be a naturally occurring nucleic acid such as DNA or RNA.

Examples of anionic surfactants include lithium dodecyl sulfate (LDS) and sodium dodecyl sulfate. The anionic surfactant is preferably lithium dodecyl sulfate (LDS), since it hardly precipitates at low temperatures.

An example of chaotropic compounds is a guanidinium salt. The guanidinium salt may be, for example, guanidinium (iso)thiocyanate (GuSCN) or guanidinium hydrochloride.

After the step (i), (ii) the lysate, an alkanol having a boiling point higher than 75°C, a chaotropic compound and silica particles are mixed to adsorb the nucleic acid on the silica particles.

To adsorb the nucleic acid on the silica particles, for example, the adsorbing liquid comprising the alkanol having a boiling point higher than 75°C, the chaotropic compound and the silica particles is added to the microtube containing the above lysate and placed on a heat block, and is mixed, then heated.

From a viewpoint of reducing the amount of time necessary from the lysis to the adsorption, the temperature at which step (i) and step (ii) are performed is a temperature higher than 75°C, and is preferably 80°C or more and more preferably 85°C or more. The temperature at which step (i) and step (ii) are performed is preferably 100°C or less, more preferably 98°C or less, and further preferably 95°C or less. The temperature at which step (i) and step (ii) are performed is, for example, 75 to 100°C, 75 to 98°C, 75 to 95°C, 80 to 100°C, 80 to 98°C, 80 to 95°C, 85 to 100°C, 85 to 98°C, or 85 to 95°C.

Setting the temperature at which step (i) and step (ii) are performed both to a temperature higher than 75°C allows to reduce the amount of time necessary for the temperature change of the liquid temperature. Therefore, it allows to reduce the amount of time necessary from the lysis of the biological sample to the adsorption of the nucleic acid. As a result, the amount of time necessary for the extraction of nucleic acid (the amount of time necessary from the lysis of the biological sample to the elution of the nucleic acid) can also be reduced.

The boiling point of the alkanol used in step (ii) is a temperature higher than 75°C, and is preferably 80°C or more and more preferably 85°C or more. The boiling point of the alkanol used in step (ii) is preferably 120°C or less, more preferably 110°C or less, and further preferably 100°C or less. The boiling point of the alkanol used in step (ii) is, for example, 75 to 120°C, 75 to 110°C, 75 to 100°C, 80 to 120°C, 80 to 110°C, 80 to 100°C, 85 to 120°C, 85 to 110°C, or 85 to 100°C.

The alkanol having a boiling point higher than 75°C is, for example, 1-propanol, ethanol, isopropanol and the like.

A specific example of the chaotropic compound is the same as the specific example of the chaotropic compound in step (i).

Examples of silica particles include particles constituted of alkyl silica, aluminum silicate (also referred to as "zeolite"), silica having an amino group, and the like. From a viewpoint of easier collection, the silica particles are preferably magnetic silica particles.

After step (ii), (iii) the silica particles having the nucleic acid adsorbed thereon are washed with a washing liquid.

The step (iii) may comprise, for example, a step of adding a first washing liquid comprising an alcohol and an anionic surfactant to the silica particles having the nucleic acid adsorbed thereon, followed by stirring, and then separating the silica particles having the nucleic acid adsorbed thereon and the first washing liquid, and a step of adding a second washing liquid comprising polyethylene glycol to the silica particles after the separation, followed by stirring, and then separating the silica particles having the nucleic acid adsorbed thereon and the second washing liquid.

The alcohol may be 2-propanol, 1-propanol or ethanol. The alcohol may also be a mixture of these.

The anionic surfactant may be, for example, sodium dodecyl sulfate (SDS), or may be lithium dodecyl sulfate. The anionic surfactant may also be a mixture of these.

The average molecular weight of polyethylene glycol may be 200 to 10000. An example of such polyethylene glycol includes polyethylene glycol 4000 (PEG4000). In the present description, the average molecular weight of polyethylene glycol means the value measured in accordance with the average molecular weight test of each macrogol (polyethylene glycol) described in the Japanese Pharmacopoeia, Sixteenth Edition.

After step (iii), (iv) the nucleic acid adsorbed on the silica particles is eluted with an eluent.

The nucleic acid can be eluted, for example, by the following method. Namely, the silica particles on which the nucleic acid is adsorbed to and the eluent are first mixed and stirred, then heated. After stirring again, the nucleic acid can be eluted by separating the silica particles and the solution.

From a viewpoint of eluting the adsorbed nucleic acid efficiently and in a short time, the temperature at which step (iv) is performed is a temperature higher than 75°C, and is preferably 80°C or more and more preferably 85°C or more. The temperature at which step (iv) is performed is preferably 100°C or less, more preferably 98°C or less, and further preferably 95°C or less. The temperature at which step (iv) is performed is, for example, 75 to 100°C, 75 to 98°C, 75 to 95°C, 80 to 100°C, 80 to 98°C, 80 to 95°C, 85 to 100°C, 85 to 98°C, or 85 to 95°C.

Setting the temperature at which step (iv) is performed to a temperature higher than 75°C similarly as the temperature at which step (ii) is performed, allows to perform the extraction of nucleic acid (from the lysis of the biological sample to the elution of the nucleic acid) without changing the heating conditions of the heater such as a heat block. Therefore, the amount of time necessary for changing the temperature inside the heater and of the solution can be reduced, and the amount of time necessary for the extraction of the nucleic acid (the amount of time necessary from the lysis of the biological sample to the elution of the nucleic acid) can further be reduced.

Setting all the temperatures at which step (i), step (ii) and step (iv) are performed to a temperature higher than 75°C allows to obtain a high-yield nucleic acid, in a short time, from a biological sample. Moreover, if the temperatures at which step (i), step (ii) and step (iv) are performed are all temperatures higher than 75°C, there may be a single heater and a plurality of heaters set at different temperatures is not required, since it is possible to perform step (i), step (ii) and step (iv) under the same range of temperature conditions.

Examples of eluents include sterile water and buffer solutions with a low salt concentration. A buffer solution with a low salt concentration is, for example, a buffer solution comprising 10 mM of tris-hydrochloric acid (Tris-HCl).

The above step (i) to step (iv) may be performed manually, or may be performed fully automatically using a nucleic acid extractor.

Each solution necessary to the method according to the present embodiment can be packaged in advance, and utilized as a nucleic acid extraction kit. Namely, the nucleic acid extraction kit comprises a first lysis solution comprising an anionic surfactant. Specific examples of anionic surfactants are the same as the specific examples of anionic surfactants in the step (i) of the method for extracting nucleic acid from a biological sample.

As the concentration in the mixed liquid of the biological sample and the first lysis solution, the concentration of the anionic surfactant is preferably 0.01 to 10 mass%, more preferably 0.1 to 5 mass%, and further preferably 0.5 to 3 mass%.

The first lysis solution may further comprise tris-hydrochloric acid (Tris-HCl) and ethylenediaminetetraacetic acid (EDTA).

The nucleic acid extraction kit further comprises a second lysis solution comprising a chaotropic compound.

Specific examples of the chaotropic compound are the same as the specific examples of the chaotropic compound in step (i) of the method for extracting nucleic acid from a biological sample.

As the concentration in the mixed liquid of the biological sample, the first lysis solution and the second lysis solution, the concentration of the chaotropic compound contained in the second lysis solution is preferably 0.01 to 5 M, more preferably 0.1 to 4.5 M, and further preferably 1 to 4 M.

The second lysis solution may further comprise tris-hydrochloric acid (Tris-HCl).

The nucleic acid extraction kit further comprises an adsorbing liquid comprising an alkanol having a boiling point higher than 75°C, a chaotropic compound and silica particles.

Specific examples and preferable aspects of the alkanol having a boiling point higher than 75°C, the chaotropic compound and the silica particles are the same as the specific examples and preferable aspects of the alkanol having a boiling point higher than 75°C, the chaotropic compound and the silica particles in the method for extracting nucleic acid from a biological sample.

As the concentration in the mixed liquid of the above lysate and adsorbing liquid, the concentration of the alkanol having a boiling point higher than 75°C is preferably 1 to 99 mass%, more preferably 10 to 90 mass%, and further preferably 20 to 70 mass%.

As the concentration in the mixed liquid of the above lysate and adsorbing liquid, the concentration of the chaotropic compound contained in the adsorbing liquid is preferably 0.01 to 5 M, more preferably 0.1 to 4 M, and further preferably 1 to 4.5 M.

The nucleic acid extraction kit may further comprise a first washing liquid, a second washing liquid and an eluent in the method for extracting nucleic acid from a biological sample.

The nucleic acid extraction kid according to the present embodiment is applicable to a wide range of applications. With the nucleic acid extraction kit according to the present embodiment, the extraction of nucleic acid from a wide range of biological materials such as bacteria, fungi and viruses contained in clinical samples such as blood, throat swabs, nasopharyngeal swabs, sputum, spinal fluid, urine, feces and saliva, is possible with the same kit. Moreover, it is possible to provide a method for extracting nucleic acid and a kit used therefor, that can reduce the amount of time necessary for the extraction compared to a general method.

### Examples

The present invention will now be described in more detail based on Examples, but the present invention is not intended to be limited to Examples, but rather it is defined in the appended claims.

The bacteria and viruses shown in the following Table 1 were used. S. pneumoniae (also referred to as "S.P") were cultivated from a culture plate, suspended in a normal saline solution and adjusted to a bacteria liquid concentration of McF#1 by measuring the turbidity, to be used as specimen (1). B. pertussis (also referred to as "B.P") were cultivated from a culture plate, suspended in a normal saline solution and adjusted to a bacteria liquid concentration of McF#1 by measuring the turbidity, to be used as specimen (2). S. cerevisiae (also referred to as "S.C") were cultivated from a culture plate, suspended in a normal saline solution and adjusted to a bacteria liquid concentration corresponding to OD = 6.0 by measuring the turbidity at a wavelength of 600 nm, to be used as specimen (3). Human adenovirus 2 (also referred to as "ADV") was infected to A549 cells and cultured, and what was recovered from the culture supernatant was used as specimen (4). Influenza A virus (subtype H3N2, also referred to as "FluA") was infected to MDCK cells and cultured, and its culture supernatant was used as specimen (5).

**[Table 1]**

| | Strain used |
|---|---|
| *S.pneumoniae,* S.P | ATCC49619 |
| *B.pertussis,* B.P | Clinical isolate |
| *S.cerevisiae,* S.C | BY611 |
| *Human Adenovirus* 2, ADV | ATCCVR-846 |
| Influenza A virus (subtype H3N2, FluA) | Clinical isolate |

As the nucleic acid extraction reagents, the solutions with the compositions shown below were prepared.
First lysis solution: 233.3 mM Tris-HCl (pH 7.5), 23.3 mM EDTA, 4.6 (w/w)% LDS
Second lysis solution: 100 mM Tris-HCl (pH 7.5), 4.25 M GuSCN
Adsorbing liquid: 47.3 (w/w)% 1-propanol, 2.5 M GuSCN, magnetic silica particles
First washing liquid: 41.0 (w/w)% 2-propanol, 1 M NaCl, 1.1 (w/w)% SDS
Second washing liquid: 9.5 (w/w)% PEG 4000, 1 M NaCl
Eluent: 10 mM Tris-HCl (pH 7.5)

### (Example 1: Extraction of nucleic acid by automated nucleic acid extractor)

Nucleic acid was extracted from S.P, B.P, S.C, ADV and FluA fully automatically using a nucleic acid extractor, by the following procedure. Namely, 3 µL of the S.P specimen (1) was added to 200 µL of a normal saline solution, to obtain biological sample A. Similarly, 2 µL of the B.P specimen (2), 3 µL of the S.C specimen (3), 3 µL of the ADV specimen (4) and 1 µL of the FluA specimen (5) were each added to 200 µL of a normal saline solution, to obtain biological samples B to E. The biological samples A to E and 150 µL of the first lysis solution were mixed in microtubes, then stirred for 7 s. Next, they were heated on a heat block at 110°C for 1 min. While continuing to heat on the heat block, 350 µL of the second lysis solution was further added and stirred for 7 s, then heated on the heat block at 110°C for 1 min. While continuing to heat on the heat block, 800 µL of the adsorbing liquid was further added, then stirred for 7 s. Subsequently, after heating on the heat block at 110°C for 1 min, they were stirred for 7 s and further heated on the heat block at 110°C for 1 min. After separating the magnetic silica particles having the nucleic acid adsorbed thereon and the solutions, 900 µL of the first washing liquid was added and stirred for 7 s. After separating again the magnetic silica particles having the nucleic acid adsorbed thereon and the solutions, 900 µL of the second washing liquid was added and stirred for 7 s. After collecting the magnetic silica particles and discarding the supernatant, 250 µL of the eluent was added and stirred for 7 s. After letting it rest on the heat block at 110°C for 2 min, they were stirred for 7 s and the magnetic silica particles having the nucleic acid adsorbed thereon and the solutions were separated, to obtain nucleic acid extract liquids I to V. The liquid temperature was monitored and it was confirmed that the liquid temperature higher than 75°C during the lysis step, the adsorption step and the elution step.

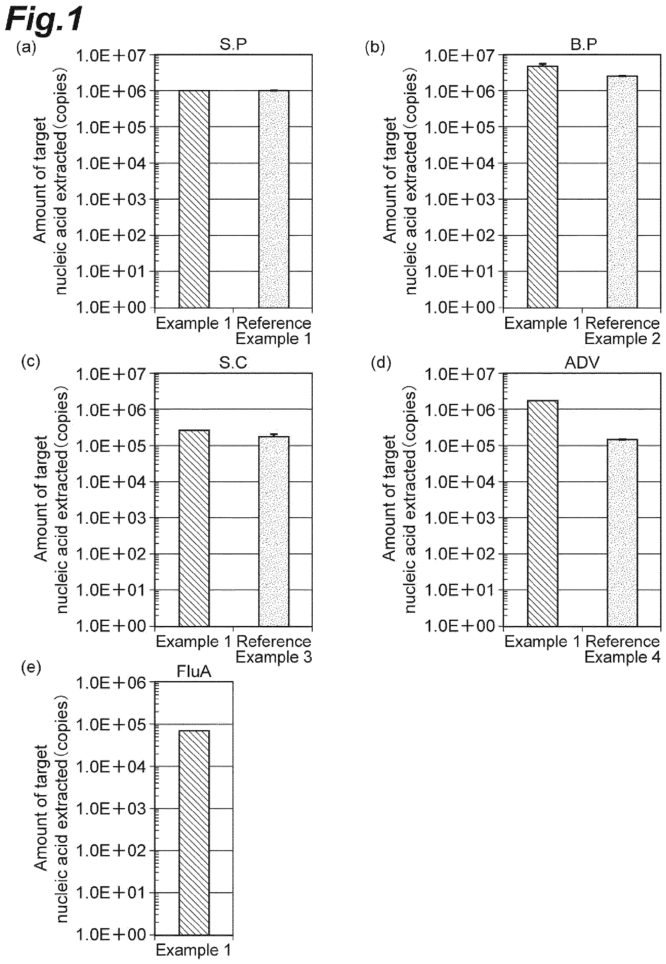

The nucleic acids were quantified by conducting a PCR reaction using the nucleic acids in the obtained nucleic acid extract liquids I to V as templates. Namely, the PCR was first performed with the reagent compositions and under the reaction conditions shown below, using the primer sets and probes shown in the following Table 2. Regarding Example 1, the same extraction and quantification were performed three times. The results of the average value and the standard error are shown in Figures 1(a) to (e).

**[Table 2]**

| target nucleic acid | Forward primer (F), Reverse primer (R) and Probe (P) (5'-3') | SEQ ID No. |
|---|---|---|
| S.pneumoniae (S.P) | (F)ACGCAATCTAGCAGATGAAGC | 1 |
| | (R)TGTTTGGTTGGTTATTCGTGC | 2 |
| | (P)TTTGCCGAAAACGCTTGATACAGGG[FAM-TAMRA] | 3 |
| B.pertussis (B.P) | (F)ATCAAGCACCGCTTTACCC | 4 |
| | (R)TTGGGAGTTCTGGTAGGTGTG | 5 |
| | (P)AATGGCAAGGCCGAACGCTTCA[FAM-TAMRA] | 6 |
| S.cerevisiae (S.C) | (F)GGACTCTGGACATGCAAGAT | 7 |
| | (R)ATACCCTTCTTAACACCTGGC | 8 |
| | (P)CCCTTCAGAGCGTTTTCTCTAAATTGATAC[FAM-BHQ1] | 9 |
| Adenovirus (ADV) | (F)GCCACCGTGGGGTTTCTAAACTT | 10 |
| | (R)GCCGCAGTGGTCTTACATGCACATC | 11 |
| | (P)TGCACCAGGCCCCGGCTCAGGTACTCCGA[FAM-TAMRA] | 12 |
| InfluenzaA (FluA) | (F)CCMAGGTCGAAACGTAYGTTCTCTCTATC | 13 |
| | (R)TGACAGRATYGGTCTTGTCTTTAGCCAYTCCA | 14 |
| | (P)ATYTCGGCTTTGAGGGGGCCTG[FAM-MGB] | 15 |

Composition of the PCR reaction solution and conditions of the PCR reaction

**Nucleic acid extract liquid I or IV**

| | |
|---|---|
| 2x Premix Ex Taq™ (Takara) | 12.5 µL |
| 10 µM forward primer | 0.5 µL |
| 10 µM reverse primer | 0.5 µL |
| 10 µM probe | 0.5 µL |
| DNase, RNase free water (Sigma) | 6 µL |
| Template | 5 µL |

| | |
|---|---|
| Heat denaturation 95°C 10 s | 1 cycle |
| Amplification [95°C 5 s -> 60°C 20 s] | 40 cycles |

**Nucleic acid extract liquid II**

| | |
|---|---|
| 2x Premix Ex Taq™ (Takara) | 10 µL |
| 10 µM forward primer | 1.8 µL |
| 10 µM reverse primer | 1.8 µL |
| 10 µM probe | 1.0 µL |
| DNase, RNase free water (Sigma) | 0.4 µL |
| Template | 5 µL |

| | |
|---|---|
| Heat denaturation 95°C 15 s | 1 cycle |
| Amplification [95°C 3 s -> 57°C 30 s] | 40 cycles |

**Nucleic acid extract liquid III**

| | |
|---|---|
| 2x Premix Ex Taq™ (Takara) | 12.5 µL |
| 50 µM forward primer | 0.25 µL |
| 50 µM reverse primer | 0.25 µL |
| 10 µM probe | 0.5 µL |
| DNase, RNase free water (Sigma) | 6.5 µL |
| Template | 5 µL |

| | |
|---|---|
| Heat denaturation 95°C 10 s | 1 cycle |
| Amplification [95°C 5 s -> 60°C 20 s] | 40 cycles |

**Nucleic acid extract liquid V**

| | |
|---|---|
| 2x QuantiTect Probe RT-PCR Master Mix (QIAGEN) | 12.5 µL |
| 50 µM forward primer | 0.3 µL |
| 50 µM reverse primer | 0.3 µL |
| 5 µM probe | 0.5 µL |
| QuantiTect RT Mix (QIAGEN) | 0.25 µL |
| 20 IU/µL Rnase Inhibitor | 0.1 µL |
| DNase, RNase free water (Sigma) | 6.05 µL |
| Template | 5 µL |

| | |
|---|---|
| Reverse transcription 50°C 30 min | 1 cycle |
| Heat denaturation 95°C 15 min | 1 cycle |
| Amplification [94°C 15 s -> 56°C 75 s] | 45 cycles |

### (Example 2: Extraction of S. pneumoniae nucleic acid in a sample from a healthy subject, by automated nucleic acid extractor)

Nucleic acid was extracted by spiking the specimen (1) in the sample from a healthy subject. First, 3 µL of specimen (1) was added to 200 µL of throat swab suspension, to obtain biological sample F. Similarly, 3 µL of specimen (1) was added to 200 µL of nasopharyngeal swab suspension, 3 µL of specimen (1) was added to 200 µL of blood serum, 3 µL of specimen (1) was added to 200 µL of blood (containing the anticoagulant EDTA-2K), 3 µL of specimen (1) was added to 200 µL of blood (containing the anticoagulant sodium citrate) and 3 µL of specimen (1) was added to 200 µL of blood (containing the anticoagulant heparin), to obtain biological samples G to K respectively. The nucleic acid extract liquids VI to X were obtained in the same way as Example 1.

In regard to the obtained nucleic acid extract liquids VI to X, nucleic acid was quantified by conducting PCR reactions the same way as Example 1.

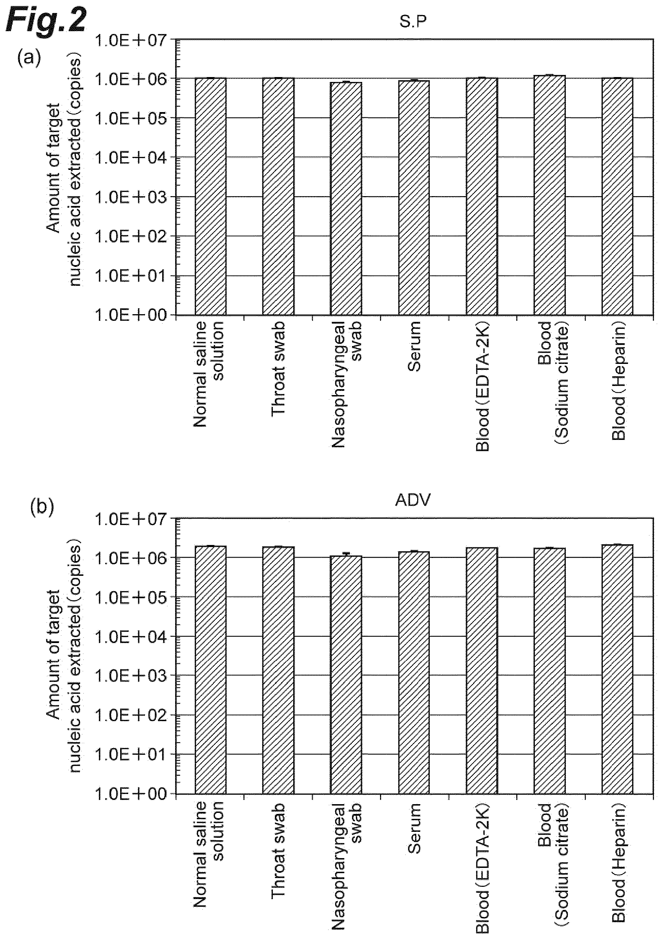

For the preparation of the biological sample H, powdered serum from healthy subject was restored with purified water and used as a sample from healthy subject, and extraction and quantification were performed three times to obtain the results of N = 3. For the preparation of the biological samples F, G, I, J and K, healthy subject samples from three donors each were used, and extraction and quantification were performed once and the results were considered as the results of N = 3. The results of N = 3 are shown in Figure 2(a) as the average value and standard error. As a control, the result of the extract liquid I in Example 1 (normal saline solution) is also shown.

### (Example 3: Extraction of adenovirus nucleic acid in a sample from healthy subject, by automated nucleic acid extractor)

Except that specimen (4) was used instead of specimen (1), the nucleic acid extract liquid was obtained and quantified by conducting a PCR reaction the same way as Example 2. The results are shown in Figure 2(b). As a control, the result of the extract liquid IV in Example 1 (normal saline solution) is also shown.

### (Example 4: Relationship between elution temperature and the recovery rate of S. pneumoniae genome DNA)

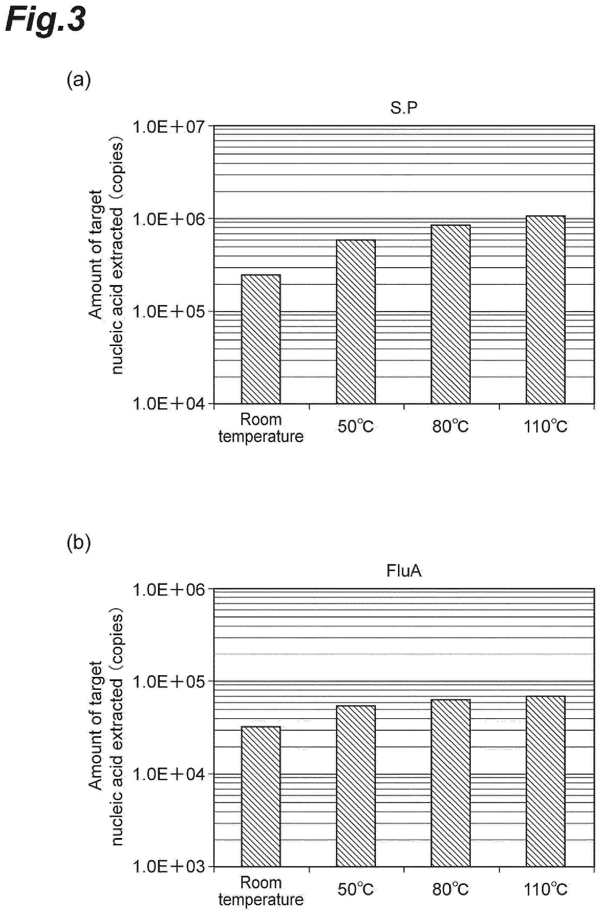

Except performing in an environment where the elution temperature was set at room temperature (25°C), 50°C, 80°C or 110°C, the extract liquid was obtained from the biological sample A of specimen (1), a PCR reaction was performed and the nucleic acid was quantified the same way as Example 1. The results are shown in Figure 3(a).

### (Example 5: Relationship between elution temperature and the recovery rate of Influenza virus A genome DNA)

Except performing in an environment where the elution temperature was set at room temperature (25°C), 50°C, 80°C or 110°C, the extract liquid was obtained from the biological sample A of specimen (1), a PCR reaction was performed and the nucleic acid was quantified the same way as Example 1. The results are shown in Figure 3(b).

### (Reference Example 1)

Using the QIAamp (trademark) DNA mini kit (by QIAGEN), nucleic acid was extracted from a biological sample containing S.P. Namely, except using specimen (1) as a sample instead of centrifuged sediment and using 10 mM Tris-HCl (pH 7.5) instead of Buffer AE or purified water, nucleic acid was extracted in accordance with the recommended protocols of the kit. The recommended protocols of the kit are Appendix D: Protocol for Bacteria, Isolation of genomic DNA from Gram-positive bacteria and Protocol: DNA purification from Tissues described in QIAamp DNA Mini and Blood Mini Handbook (Fifth edition). Regarding the nucleic acid extracted using the QIAamp (trademark) DNA mini kit (by QIAGEN), nucleic acid was quantified by conducting a PCR reaction the same way as Example 1. The result is shown in Figure 1(a).

### (Reference Example 2)

Using the QIAamp (trademark) DNA mini kit (by QIAGEN), nucleic acid was extracted from a biological sample containing B.P. Namely, except using specimen (2) as a sample instead of centrifuged sediment, performing the protease K treatment for 10 min and using 10 mM Tris-HCl (pH 7.5) instead of Buffer AE or purified water, nucleic acid was extracted in accordance with the recommended protocols of the kit. The recommended protocols of the kit are Appendix D: Protocol for Bacteria, Isolation of genomic DNA from bacterial plate cultures and Protocol: DNA purification from Tissues described in QIAamp DNA Mini and Blood Mini Handbook (Fifth edition). In regard to the extracted nucleic acid, the nucleic acid was quantified the same way as in Reference Example 1. The result is shown in Figure 1(b).

### (Reference Example 3)

Using the QIAamp (trademark) DNA mini kit (by QIAGEN), nucleic acid was extracted from a biological sample containing S.C. Namely, except using specimen (3) as a sample instead of centrifuged sediment for each, performing the protease K treatment for 10 min and using 10 mM Tris-HCl (pH 7.5) instead of Buffer AE or purified water, nucleic acid was extracted in accordance with the recommended protocols of the kit. The recommended protocols of the kit are Appendix E: Protocol for Yeast and Protocol: DNA purification from Tissues described in QIAamp DNA Mini and Blood Mini Handbook (Fifth edition). In regard to the extracted nucleic acid, the nucleic acid was quantified the same way as Reference Example 1. The result is shown in Figure 1(c).

### (Reference Example 4)

Using the QIAamp (trademark) MinElute Virus Spin kit (by QIAGEN), nucleic acid was extracted from a biological sample containing ADV Namely, except using 3 µL of specimen (4) in 200 µL of normal saline solution instead of blood plasma or blood serum described in the recommended protocol of the kit, nucleic acid was extracted in accordance with the recommended protocol of the kit. The recommended protocol of the kit is Protocol: Purification of Viral Nucleic Acids from Plasma or Serum described in QIAamp (trademark) MinElute Virus Spin Handbook (2010 edition).

Regarding the nucleic acid extracted using the QIAamp (trademark) MinElute Virus Spin kit (by QIAGEN), it was quantified by conducting a PCR reaction the same way as Example 1. The result is shown in Figure 1(d).

In regard to Examples 1 to 3 and Reference Examples 1 to 4, the amount of time it took for the extraction are shown below in Table 3.

**[Table 3]**

| | Examples 1 to 3 | Reference Example 1 | Reference Example 2 | Reference Example 3 | Reference Example 4 |
|---|---|---|---|---|---|
| Extraction time (min) | 14 | 95 | 40 | 75 | 40 |

## Claims

1. A method for extracting nucleic acid from a biological sample, comprising steps of:
(i) mixing the biological sample, lithium dodecyl sulfate and a chaotropic compound to obtain a lysate;
(ii) mixing the lysate, 1-propanol, a chaotropic compound and silica particles to adsorb the nucleic acid on the silica particles;
(iii) washing the silica particles having the nucleic acid adsorbed thereon with a washing liquid; and
(iv) eluting the nucleic acid adsorbed on the silica particles with an eluent,
wherein at least steps (i) and (ii) are performed at a temperature higher than 75°C.

2. The method according to claim 1, wherein step (iv) is performed at a temperature higher than 75°C.

3. The method according to claim 1 or 2, wherein the chaotropic compound is a guanidinium salt.

4. A nucleic acid extraction kit comprising:
a first lysis solution comprising an lithium dodecyl sulfate;
a second lysis solution comprising a chaotropic compound; and
an adsorbing liquid comprising 1-propanol, a chaotropic compound and silica particles.

5. The kit according to claim 4, wherein the chaotropic compound is a guanidinium salt.

## Patentansprüche

1. Verfahren zur Extraktion von Nukleinsäure aus einer biologischen Probe mit den Schritten:
(i) Mischen der biologischen Probe, von Lithiumdodecylsulfat und einer chaotropen Verbindung, um ein Lysat zu erhalten;
(ii) Mischen des Lysats, von 1-Propanol, einer chaotropen Verbindung und Siliziumdioxidteilchen, um die Nukleinsäure an den Siliziumdioxidteilchen zu adsorbieren;
(iii) Waschen der Siliziumdioxidteilchen mit der daran adsorbierten Nucleinsäure mit einer Waschflüssigkeit; und
(iv) Eluieren der an den Siliziumdioxidteilchen adsorbierten Nucleinsäure mit einem Elutionsmittel,
wobei mindestens die Schritte (i) und (ii) bei einer Temperatur über 75°C durchgeführt werden.

2. Verfahren nach Anspruch 1, bei dem Schritt (iv) bei einer Temperatur von mehr als 75 °C durchgeführt wird.

3. Verfahren nach Anspruch 1 oder 2, bei dem die chaotrope Verbindung ein Guanidiniumsalz ist.

4. Nukleinsäure-Extraktionskit, umfassend:
eine erste Lyselösung, die ein Lithiumdodecylsulfat umfasst;
eine zweite Lyselösung, die eine chaotrope Verbindung umfasst; und
eine adsorbierende Flüssigkeit, umfassend 1-Propanol, eine chaotrope Verbindung und Siliziumdioxidteilchen.

5. Kit nach Anspruch 4, bei der die chaotrope Verbindung ein Guanidiniumsalz ist.

## Revendications

1. Procédé d'extraction d'acide nucléique à partir d'un échantillon biologique, comprenant les étapes consistant à :
(i) mélanger l'échantillon biologique, du dodécyl-sulfate de lithium et un composé chaotropique pour obtenir un lysat ;
(ii) mélanger le lysat, du 1-propanol, un composé chaotropique et des particules de silice pour adsorber l'acide nucléique sur les particules de silice ;
(iii) laver les particules de silice sur lesquelles l'acide nucléique est adsorbé avec un liquide de lavage ; et
(iv) éluer l'acide nucléique adsorbé sur les particules de silice avec un éluant,
dans lequel au moins les étapes (i) et (ii) sont effectuées à une température supérieure à 75°C.

2. Procédé selon la revendication 1, dans lequel l'étape (iv) est effectuée à une température supérieure à 75°C.

3. Procédé selon la revendication 1 ou 2, dans lequel le composé chaotropique est un sel de guanidinium.

4. Kit d'extraction d'acide nucléique comprenant :
une première solution de lyse comprenant du dodécyl-sulfate de lithium ;
une deuxième solution de lyse comprenant un composé chaotropique ; et
un liquide adsorbant comprenant du 1-propanol, un composé chaotropique et des particules de silice.

5. Kit selon la revendication 4, dans lequel le composé chaotropique est un sel de guanidinium.
